# EUROPEAN PATENT APPLICATION

(11) **EP 0 641 568 A1**
(43) Date of publication of application: **08.03.1995**
(21) Application number: 93114276.4
(22) Date of filing: 06.09.1993
(51) Int. Cl.: A61K 39/118, A61K 39/02

(54) **Chlamydia antigen, process for its manufacture and its use**

(71) Applicant: Brade, Helmut, Dr. med., D-23829 Kükels (DE)
(72) Inventor: Brade, Helmut, Dr. med., D-23829 Kükels (DE)
(74) Representative: ter Meer, Nicolaus, Dipl.-Chem., Dr.

(57) **Abstract**

Ladder-like banding pattern antigen (LLBPA) characterized in that it is resistant against proteolytic digestion, is resistant to heat (70°C, 1 h) at neutral pH is extracted by phenol-water extraction preferably into the water-phase, gives rise to a regular ladder-like banding pattern upon sodium dodecylsulfate polyacrylamide-gel electrophoresis (SDS-PAGE) as detected by silver-staining the gel or by blotting to nitrocellulose followed by staining with labelled antibodies, and induces antibodies upon immunization or infection, processes for its manufacture and its use in the diagnosis, prevention and treatment of human and animal infections.

## Description

The present invention relates to ladder-like banding pattern antigens (abbreviated in the following as LLBPA), processes for their manufacture and their use in the diagnosis and therapy of human and animal infections.

*Chlamydia psittaci, C. pneumoniae* and *C. trachomatis* are the three species of the genus *Chlamydia* which are pathogenic obligatory intracellular parasites causing diseases in animals and humans. More than 300 million of people suffer world wide from trachoma, a chronic infection of the eye which leads in the final stages to blindness. A similar number of patients have sexually transmitted diseases of the genital tract which may result in infertility, particularly in woman (chronic salpingitis). *C. pneumoniae* causes a broad spectrum of diseases of the respiratory tract and has been reported as a potential cofactor in the pathogenesis of coronary heart disease and myocardial infarction.

Although chlamydiae are sensitive to antibiotics, antibiotic treatment often fails to eradicate the infectious agent due to the fact that chlamydiae are obligatory intracellular parasites which can persist in the host without clinical signs of a disease. In addition, it is desirable to prevent chlamydial infections.

The problem of the present invention therefore is the provision of new antigens which may be used in the diagnosis and therapy of human and animal infections, preferably for the manufacture of reagents for the diagnosis and pharmaceutical compositions for the prevention and treatment of human and animal chlamydial infections.

It has been found that this problem can be solved by means of a new antigenic material, i.e. a ladder-like banding pattern antigen (LLBPA), which has been derived from cultures of specific microorganisms.

The ladder-like banding pattern antigen (LLBPA) of the present invention is characterized in that it is resistant against proteolytic digestion, is resistant to heat (70°C, 1 h) at neutral pH, is extracted by phenol-water extraction preferably into the water-phase, gives rise to a regular ladder-like banding pattern upon sodium dodecylsulfate polyacrylamide-gel electrophoresis (SDS-PAGE) as detected by silver-staining the gel or by blotting to nitrocellulose followed by staining with labelled antibodies, and induces antibodies upon immunization or infection.

Specifically the antigen is resistant against proteolytic digestion by trypsin or proteinase K.

The antigenic material of the present invention is characterized by a specific pattern upon sodium dodecylsulfate polyacrylamide-gel electrophoresis, which is termed as "ladder-like banding pattern" and is shown in the drawings herewith enclosed. In the drawings:

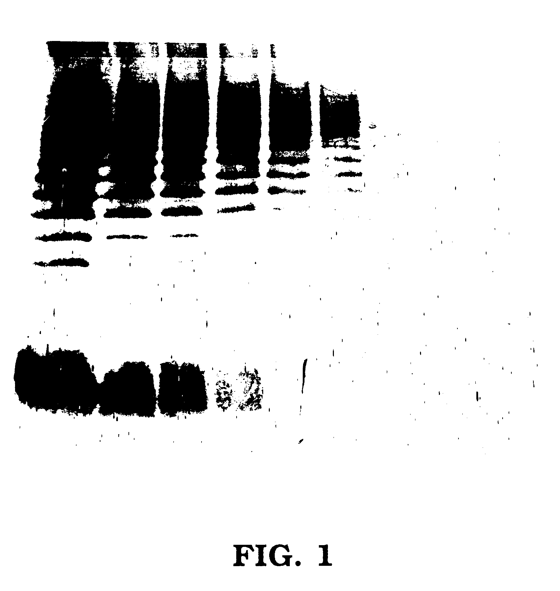

- **Fig. 1**: shows the Western-blot of LLBPA using rabbit antiserum. LLBPA from partially purified elementary bodies of *C. psittaci* 6BC according to example 1 (two-fold dilutions from 50 µg to 200 ng) were digested with proteinase K, separated by SDS-PAGE (15%), blotted to nitrocellulose, and developed with rabbit antiserum described in example 3,

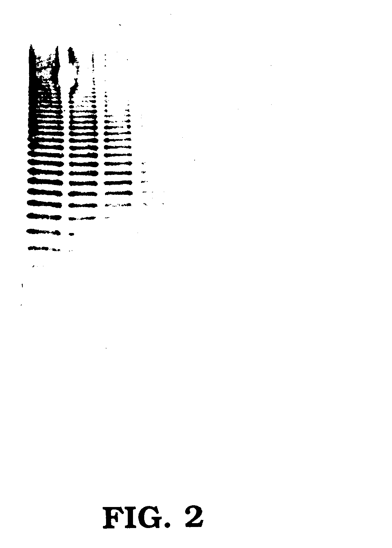

- **Fig. 2**: shows the Western-blot of LLBPA using murine monoclonal antibody. LLBPA from partially purified elementary bodies of *C. psittaci* 6BC according to example 1 (two-fold dilutions from 25 µg to 400 ng) were digested with proteinase K, separated by SDS-PAGE (10%), blotted to nitrocellulose, and developed with the monoclonal antibody described in example 4, and

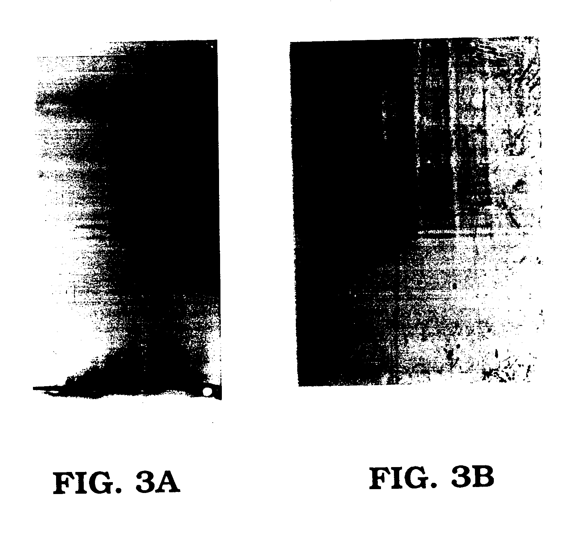

- **Fig. 3A and B**: show the Western-blot of LLBPA using rabbit antiserum. LLBPA from *C. trachomatis* serotype L2 culture supernatants according to example 2 (two-fold dilutions of amounts equivalent from 300 to 20 cm³ of infected monolayer) were digested with proteinase K, separated by SDS-PAGE (10%), blotted to nitrocellulose, and either silver-stained (A) or developed with rabbit antiserum (B) as described in example 5.

The ladder-like banding pattern antigen of the present invention can be obtained from cultures of microorganisms such as from cultures of *Chlamydia* and may be obtained in a similar way from *Neisseria*, *Bacteroides*, *Acinetobacter*, *Haemophilus* and *Bordetella* in embryonated eggs, tissue culture cells or appropriate artificial media, more preferably from cultures of *Chlamydia psittaci*, *Chlamydia pneumoniae*, *Chlamydia trachomatis*, *Neisseria meningitidis*, *Neisseria gonorrhoeae*, *Bacteroides fragilis*, *Acinetobacter calcoaceticus*, *Haemophilus influenzae* and *Bordetella* pertussis, still more preferably from cultures of *Chlamydia psittaci* 6BC or *Chlamydia trachomatis* serotype L2 or E. Such microorganism strains and specifically chlamydial strains are known and can be obtained from culture collections such as the American Type Culture Collection (ATCC) or isolated from patients.

Preferred examples of the microorganisms used according to the present invention have been deposited with the American Type Culture Collection (ATCC) under the following numbers:
*C. psittaci* strain 6BC, ATCC VR-125
*C. trachomatis* serotype L2 strain 434, ATCC VR-902
*C. trachomatis* serotype E strain BOUR, ATCC VR-948B.

The subject matter of the present invention furtheron is a process for the manufacture of the ladder-like banding pattern antigen which comprises growing said microorganism strains and specifically *Chlamydia psittaci* 6BC in embryonated eggs, killing the eggs preferably with phenol and homogenizing the eggs preferably in saline and lyophilizing the product, washing the dry product with an organic solvent, such as ethanol, acetone and ether, digesting the dry product preferably after suspension in saline with trypsine and dialyzing it against water, centrifugating and ultracentrifugating the supernatant, lyophilizing the sediment obtained containing partially purified elementary bodies, subjecting said elementary bodies to a phenol-water extraction, dialyzing the water-phase against water and lyophilizing the antigen obtained.

According to a further embodiment of the present invention the process for the manufacture of the antigens under consideration comprises growing said microorganism strains and preferably *Chlamydia trachomatis* serotype L2 or E in tissue cells, preferably tissue cells L929, killing the cells preferably with phenol, centrifuging the mixture of cells, cell debris and said microorganisms, ultracentifuging the supernatant, dialyzing the supernatant obtained, digesting it with trypsin, dialyzing again and lyophilizing the product, subjecting the dry product to a phenol-water extraction, dialyzing the water-phase against water and lyophilizing the desired antigen.

The above tissue cells L929 are known to the man of the art and can be obtained from every culture collection. Furtheron the tissue cells L929 may be substituted by other tissue cells, such as McCoy, HeLa and LLCMK₂-cells.

The present invention furtheron comprises the use of the antigen defined above in the diagnosis, prevention and treatment of Chlamydia infections and more specifically for the manufacture of reagents for the diagnosis and of pharmaceutical compositions for the prevention and treatment of human and animal infections and preferably chlamydial infections.

The antigen can be used
- to detect antibodies,
- as a competitor in antigen-detection assays and
- as an immunogen to induce poly- or monoclonal antibodies and making use of such antibodies in diagnosis, prevention or treatment of human and animal infections, specifically chlamydial infections.

The invention is described in the following more in detail with reference to the following examples.

### Example 1 (Isolation of LLBPA from yolk sac-grown elementary bodies of C. psittaci strain 6BC):

*C. psittaci* strain 6BC was grown in embryonated eggs. Yolk-sacs from 100 eggs were combined, killed with phenol (0.5% final concentration), homogenized in saline (1,000 ml) and lyophilized. The dry product was washed with ethanol, acetone, and ether (200 ml each), dried, suspended in saline (500 ml), digested with trypsin (1%, 37°C, 2 h), and dialyzed against water After centrifugation (2,860 g for 30 min), the sediment was lyophilized, the supernatant was ultracentifuged (100,000 g for 4 h), yielding a supernatant and a sediment. The latter contained partially purified elementary bodies which were subjected to hot phenol-water extraction (2) yielding a water- and phenol-phase. The water-phase was dialyzed against water, lyophilized, and aliquots were analyzed by SDS-PAGE followed by western-blot (Fig. 1 and 2).

### Example 2 (Isolation of LLBPA from particle-free supernatants of tissue cultures infected with C. trachomatis serotype L2):

*C. trachomatis* serotype L2 was grown in L929 cells in 6,000 cm³ plastic multi-trays. Fourty-eight hours after infection, phenol was added (0.5% final concentration) followed by shaking over night. The mixture of cells, cell debris and chlamydiae was centrifuged (2,860 g for 30 min). The supernatant was centrifuged again (20,000 g for 4 h) whereby partially purified elementary bodies were obtained in the resulting sediment. The supernatant was dialyzed, digested with trypsin (1%, 37°C, 2 h), dialyzed again, and lyophilized. The dry product was extracted with phenol-water, yielding a water- and phenol-phase. The water-phase was dialyzed and aliquots were analyzed by SDS-PAGE followed by western-blot (Fig. 3).

### Example 3 (Preparation of rabbit antisera against LLBPA from yolk sac-grown elementary bodies of C. psittaci strain 6BC):

Partially purified elementary bodies of *C. psittaci* 6BC as described in example 1 were used to immunize rabbits. The animals received within 160 days 8 i.v. injections of 50 to 400 µg of bacteria suspended in saline and were then exsanguinated. Serial dilutions of the antiserum obtained were tested by western blot using the antigen described in example 1. The result is shown in Fig. 1.

### Example 4 (Preparation of murine monoclonal antibodies against LLBPA from yolk sac-grown elementary bodies of C. psittaci strain 6BC):

Partially purified elementary bodies of *C. psittaci* 6BC as described in example 1 were used to immunize mice. BALB/c mice were injected with 50 µg of 6BC elementary bodies in Freund's complete adjuvant. On day 0, four aliquots of 50 µg each were injected s.c. and 50 µg were applicated i.p. On day 28, mice received a single i.p. injection of 50 µg in Freund's incomplete adjuvant. Eight days later, the mice were bled and tested for the presence of antibodies by western-blot using the antigen described in example 1. The mouse with the highest titer received three injections of 200 µg each on days 56, 57 and 58. The first injection was i.v., the other two were i.p. Two days after the last injection, the animal was exsanguinated and spleenocytes were fused with mouse myeloma cells X63Ag8. Screening was by western-blot using the antigen described in example 1. Relevant hybridomas were cloned twice by limiting dilution, adapted to serum-free medium and isotyped. One monoclonal antibody exhibited a reaction pattern shown in Fig. 2.

The mouse myeloma cells X63Ag8 used for the fusion can be substituted by a number of cell lines from different animals, such as mouse, guinea-pig, hamster and rat. Such cell lines are known to the man skilled in the art and can be obtained from any culture collection.

### Example 5 (Preparation of rabbit antisera against LLBPA from C. trachomatis strain L2 grown in tissue cultures):

Partially purified elementary bodies of C. trachomatis L2 as described in example 2 were used to immunize rabbits. The animals received within 160 days 8 i.v. injections of 50 to 400 µg of bacteria suspended in saline and were then exsanguinated. Serial dilutions of the antiserum obtained were tested by western blot using the antigen described in example 2. The result is shown in Fig. 3.

## Claims

1. Ladder-like banding pattern antigen (LLBPA) **characterized in that** it is resistant against proteolytic digestion,
it is resistant to heat (70°C, 1 h) at neutral pH,
it is extracted by phenol-water extraction preferably into the water-phase, it gives rise to a regular ladder-like banding pattern upon sodium dodecylsulfate polyacrylamide-gel electrophoresis (SDS-PAGE) as detected by silver-staining the gel or by blotting to nitrocellulose followed by staining with labelled antibodies, and
it induces antibodies upon immunization or infection.

2. The antigen according to claim 1, **characterized in that** it is resistant against proteolytic digestion by trypsin or proteinase K.

3. The antigen according to claims 1 or 2, **characterized in that** it has been derived from cultures of *Chlamydia, Neisseria*, *Bacteroides*, *Acinetobacter*, *Haemophilus* and *Bordetella* in embryonated eggs, tissue culture cells or appropriate artificial media.

4. The antigen according to claims 1 or 2, **characterized in that** it has been derived from cultures of *Chlamydia psittaci*, *Chlamydia pneumoniae*, *Chlamydia trachomatis*, *Neisseria meningitidis*, *Neisseria gonorrhoeae, Bacteroides fragilis, Acinetobacter calcoaceticus, Haemophilus influenzae* and *Bordetella pertussis.*

5. The antigen according to claims 1 or 2, **characterized in that** it has been derived from cultures of *Chlamydia psittaci* 6BC or *Chlamydia trachomatis* serotype L2 or E.

6. A process for the manufacture of the antigen according to claims 1 to 5 **characterized by** growing said microorganism strains in embryonated eggs, killing and homogenizing the eggs, washing the lyophilized dry product with organic solvents, digesting it with trypsin and dialyzing it against water, centrifugating and ultracentrifugating the supernatant, lyophilizing the sediment obtained containing partially purified elementary bodies, subjecting said elementary bodies to a phenol-water extraction, dialyzing the water-phase against water and lyophilizing the antigen obtained.

7. The process for the manufacture of the antigen according to claim 6 **characterized by** growing *Chlamydia psittaci* 6BC strain in embryonated eggs, killing with phenol and homogenizing the eggs in saline and lyophilizing the product, washing the dry product with ethanol, acetone and ether, digesting the dried product after suspension in saline with trypsin and dialyzing it against water, centrifugating and ultracentrifugating the supernatant, lyophilizing the sediment obtained by centrifugation containing partially purified elementary bodies, subjecting said elementary bodies to a hot phenol-water extraction, dialyzing the water-phase against water and lyophilizing the antigen obtained.

8. A process for the manufacture of the antigen according to claims 1 to 5 **characterized by** growing said microorganism strains in tissue cells, killing the cells, centrifuging the mixture of cells, cell debris and said microorganisms, centrifuging the supernatant, dialyzing the supernatant obtained, digesting it with trypsin, dialyzing again and lyophilizing the product, subjecting the dry product to a phenol-water extraction, dialyzing the water-phase against water and lyophilizing the antigen obtained.

9. The process for the manufacture of the antigen according to claim 8 **characterized by** growing *Chlamydia trachomatis* serotype L2 or E in tissue cells L929, killing the cells with phenol, centrifuging the mixture of cells, cell debris and said microorganisms, ultracentrifuging the supernatant, dialyzing the supernatant obtained, digesting it with trypsin, dialyzing again and lyophilizing the product, subjecting the dry product to a phenol-water extraction, dialyzing the water-phase against water and lyophilizing the antigen obtained.

10. The use of the antigen according to any one of claims 1 to 5 in the diagnosis, prevention and treatment of microorganism infections.

11. The use of the antigen according to any one of claims 1 to 5 for the manufacture of reagents for the diagnosis and of pharmaceutical compositions for the prevention and treatment of chlamydial infections.
